# EUROPÄISCHE PATENTSCHRIFT

(11) **EP 1 413 298 B1**
(45) Veröffentlichungstag und Bekanntmachung des Hinweises auf die Patenterteilung: **21.11.2007**
(21) Anmeldenummer: 02021148.8
(22) Anmeldetag: 24.09.2002
(51) Int. Cl.: A61K 9/50, A61K 8/00

(54) **Verfahren zur Herstellung von Makrokapseln**
Process for the manufacture of macrocapsules
Procédé pour la préparation de macrocapsules

(43) Veröffentlichungstag der Anmeldung: 28.04.2004
(73) Patentinhaber: Cognis IP Management GmbH, 40589 Düsseldorf (DE)
(72) Erfinder: Viladot Petit, Josef-Lluis, Dr., 08018 Barcelona (ES); Wollmann, Gerhard, Dr., 40923 Hilden (DE); Müller, Ralf, 60594 Frankfurt (DE)

(56) Entgegenhaltungen:
- EP-A- 1 064 911
- EP-A- 1 064 913
- WO-A-91/07951
- WO-A-93/24112
- WO-A-99/65463
- US-A- 5 550 178

## Beschreibung

**Gebiet der Erfindung**

Die Erfindung befindet sich auf dem Gebiet der kosmetischen und pharmazeutischen Zubereitungen und betrifft ein Verfahren zur Herstellung von Makrokapseln durch Vertropfung in ein Fällbad.

### Stand der Technik

Unter dem Begriff "Makrokapsel" werden vom Fachmann sphärische Aggregate mit einem Durchmesser im Bereich von etwa 0,1 bis etwa 5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Die mikroskopisch kleinen Kapseln, auch Nanokapseln genannt, lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinghydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Kapseln des Stands der Technik sind folgende Handelsprodukte (in Klammern angegeben ist jeweils das Hüllmaterial) : *Hallcrest Microcapsules™* (Gelatine, Gummi Arabicum), *Coletica Thalaspheres*^{®} (maritimes Collagen), *Lipotec Millicapseln™* (Alginsäure, Agar-Agar), *Induchem Unispheres*^{®} (Lactose, mikrokristalline Cellulose, Hydroxypropylmethylcellulose); *Unicerin*^{®}*C30* (Lactose, mikrokristalline Cellulose, Hydroxypropylmethylcellulose), *Kobo Glycospheres*^{®} (modifizierte Stärke, Fettsäureester, Phospholipide), *Softspheres*^{®} (modifiziertes Agar-Agar) und *Kuhs Probiol Nanospheres*^{®} (Phospholipide) sowie *Primaspheres*^{®} und *Primasponges*^{®} (Chitosan, Alginate) und *Primasys*^{®} (Phospholipide).

Chitosankapseln und Verfahren zu ihrer Herstellung sind Gegenstand früherer Patenanmeldungen der Patentanmelderin **[**WO 01/01926 A1**,** WO 01/01927A1**,** WO 01/01928 A1**,** WO 01/01929A1**] .** Kapseln, bestehend aus einer Hüllmembran und einer die Wirkstoffe enthaltenden Matrix, können danach beispielsweise erhalten werden, indem man
(a1) aus Gelbildnern, Chitosanen und Wirkstoffen eine Matrix zubereitet,
(a2) gegebenenfalls die Matrix in einer Ölphase dispergiert,
(a3) die dispergierte Matrix mit wässrigen Lösungen anionischer Polymere behandelt und gegebenenfalls dabei die Ölphase entfernt.
oder
(b1) aus Gelbildnern, anionischen Polymeren und Wirkstoffen eine Matrix zubereitet,
(b2) gegebenenfalls die Matrix in einer Ölphase dispergiert,
(b3) die dispergierte Matrix mit wässrigen Chitosanlösungen behandelt und gegebenenfalls dabei die Ölphase entfernt.
oder
(c 1) wässrige Wirkstoffzubereitungen mit Ölkörpern in Gegenwart von Emulgatoren zu O/W-Emulsionen verarbeitet,
(c2) die so erhaltenen Emulsionen mit wässrigen Lösungen anionischer Polymere behandelt,
(c3) die so erhaltene Matrix mit wässrigen Chitosanlösungen in Kontakt bringt und
(c4) die so erhaltenen Verkapselungsprodukte von der wässrigen Phase abtrennt.

Die so erhaltenen Kapseln weisen jedoch in der Regel besonders kleine Durchmesser auf, d.h. insbesondere Gebilde, die einen mittleren Durchmesser oberhalb von 1 mm aufweisen, lassen sich auf diesem Wege nicht stabil herstellen.

Verkapselte Wirkstoffe sind auch aus anderen Druckschriften bekannt. So offenbart beispielsweise die WO 99/065463 A1 (Ben Gurion University) Systeme zur verzögerten Freisetzung von Wirkstoffen, die aus einer porösen Matrix und dem darin enthaltenen Wirkstoff bestehen. Die Systeme werden durch Vertropfung einer Alginatmatrix in ein Calciumchloridbad erhalten. Gegenstand der US 5,550,178 (VivoRx) ist ein Verfahren zur Verkapselung biologischer Wirkstoffe, bei dem die Hülle durch Vernetzung mit einem Polysaccharid oder Polyanion. Aus der WO 93/024112 A1 (Clover) ist die Mikroverkapselung von Zellen bekannt, bei denen diese in einem festen Kern aus Alginaten suspendiert vorliegen. Schließlich wird in der WO 91/007951 A1 die Verkapselung von Langerhalszellen in einer Alginat/PLL-Matrix vorgeschlagen.

Die Aufgabe der vorliegenden Erfindung hat folglich darin bestanden, neue Kapseln für die Aufnahme von Wirkstoffen zur Verfügung zu stellen, die frei von den Nachteilen des Stands der Technik sind und insbesondere einen mittleren Durchmesser im Bereich von 0,1 bis 5, vorzugsweise 1 bis 4 und insbesondere 1,5 bis 2 mm aufweisen, und sich dabei sowohl in tensidischer Umgebung als auch bei Temperaturlagerung als beständig erweisen. Eine zusätzliche Aufgabe hat ferner darin bestanden, die zu verkapselnden Inhaltsstoffe vollständig, d.h. praktisch rückstandsfrei einzuhüllen, beispielsweise im Hinblick auf die Entfärbung von Flüssigkeiten.

### Beschreibung der Erfindung

Gegenstand der Erfindung ist ein Verfahren zur kontinuierlichen Herstellung von Makrokapseln bestehend aus einer Hüllmembran und einem darin eingeschlossenen Wirkstoff, speziell solche mit einem mittleren Durchmesser im Bereich von 0,1 bis 5, vorzugsweise 1 bis 4 und insbesondere 1,5 bis 2 mm, bei dem man
(a1) einer wässrigen Zubereitung enthaltend mindestens eine polyanionische Verbindung und mindestens einen Wirkstoff mit Hilfe einer oszillierenden Membran eine Schwingung aufprägt und durch mindestens eine Düse vertropft und
(a2) die so erhaltenen Tropfen in ein Fällbad enthaltend mindestens eine polykationische Verbindung einbringt,
oder
(b1) einer wässrigen Zubereitung enthaltend mindestens eine polykationische Verbindung und mindestens einen Wirkstoff mit Hilfe einer oszillierenden Membran eine Schwingung aufprägt und durch mindestens eine Düse vertropft und
(b2) die so erhaltenen Tropfen in ein Fällbad enthaltend mindestens eine polyanionische Verbindung einbringt.

Die so erhaltenen Makrokapseln, die durch spontane Hüllbildung zwischen den gegensätzlich geladenen polyionischen Verbindungen entstehen, zeichnen sich insbesondere dadurch aus, dass sie trotz ihres erheblichen Durchmessers auch bei Temperaturbelastung und in Gegenwart von Tensiden stabil sind. Der Einschluss der zu verkapselnden Wirkstoffe erfolgt spontan und ist vollständig, was beispielsweise daran erkannt werden kann, dass bei der Verkapselung von löslichen Farbstoffpigmenten keine Verfärbung der Fällbäder auftritt. Demzufolge ist die Erfindung auch zur Entfärbung von Farbstofflösungen geeignet.

### Vertropfungsanlage

Die Vertropfung von Flüssigkeiten durch Aufprägen einer Schwingung, die mit Hilfe einer oszillierenden Membran erzeugt, ist bereits für die Verarbeitung von synthetischen Wachsen, Harzen sowie niedrigviskosen Polyestern bekannt, wenngleich zu diesem Zweck Schmelzen eingesetzt werden, denen zur Verfestigung dann ein Kühlmittel entgegengeleitet wird. Entsprechende Bauteile werden beispielsweise von der Firma Rieter-Automatik GmbH unter Bezeichnung "Droppo Line" für vertrieben. Der Tropfenabriss erfolgt durch die erhöhte Scherwirkung beim Zurückschwingen der Membran. Im Sinne der Erfindung wird die Oszillationsfrequenz so gewählt, dass die üblicherweise beim Vertropfen entstehenden Sekundärtropfen in die Primärtropfen einbezogen werden, wodurch ein regelmäßiges Tropfbild mit nahezu gleichförmiger Tröpfchengrößenverteilung sichergestellt wird. Die Schwingungsfrequenz der Membranen liegt typischerweise im Bereich von 100 bis 1000 und vorzugsweise 500 bis 800 Hz.

Die Vertropfung selbst kann beispielsweise durch eine Einzeldüse oder durch eine Düsenplatte mit 10 bis 500, vorzugsweise 50 bis 100 Öffnungen erfolgen. Beim Einsatz einer Einzeldüse kann wiederum zwischen einer Einstoff- und einer Mehrstoffdüse unterschieden werden. Während bei der vorzugsweise auswechselbaren Einstoffstoffdüse sowohl die polyanionische bzw. polykationische Komponente als auch der zu verkapselnde Wirkstoff sowie gegebenenfalls weitere Inhaltsstoffe, wie z.B. Emulgatoren, Viskositätsregulatoren oder pH-Additive als Mischung in das Fällbad gegeben werden, erlaubt die Mehrstoffdüse die Vermischung der Einsatzstoffe, also z.B. der Polyanionverbindung und des Wirkstoffs in der Düse. Beim Einsatz einer Einlochdüse - sei es eine Ein- oder Mehrstoffdüse - ist der Einlaufkegel der Düse vorzugsweise so gestaltet, dass ein nahezu druckverlustfreier Einlauf entsteht. Mehrstoffdüsen bestehen aus einem Kernrohr und einem koaxial dazu angeordneten Außenrohr, welches in der Regel um das 0,1 bis 0,3fache des Durchmessers des Kernrohres lang und am Ende schräg angefräst ist. Es hat sich dabei als vorteilhaft erwiesen, wenn das Hüllmaterial durch das Außen- und der zu verkapselnde Wirkstoff durch das Kernrohr strömt. In beiden Fällen weisen die Düsen vorzugsweise Öffnungen mit einem Durchmesser im Bereich von 0,2 bis 2, vorzugsweise 0,3 bis 0,8 mm auf. Für die kontinuierliche Herstellung der Makrokapseln hat es sich als vorteilhaft erwiesen, anstelle der Einzeldüsen Düsenplatten einzusetzen, die Öffnungen mit einem Durchmesser im Bereich von 0,3 bis 1, vorzugsweise 0,5 bis 0,8 mm aufweisen und so geformt sind, dass in allen Öffnungen praktisch der selbe Druck herrscht.

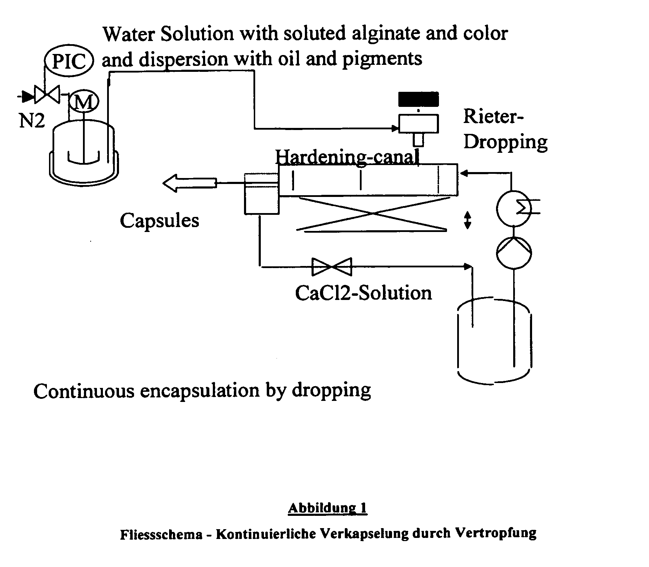

Grundsätzlich kann die Vertropfung in ein Fällbad erfolgen, welches als Rührbehälter oder Kessel ausgelegt ist. Hierbei besteht jedoch die Gefahr, dass sich Kapseln treffen und miteinander verkleben. Weiterhin können während des Rührens Kapseln wieder zerstört und ihr Inhalt freigesetzt werden; schließlich führt der Rührvorgang durch Eintrag von Energie auch zu einem unerwünschten Temperaturanstieg, so dass die Durchsatzmengen begrenzt sind. Um diese Nachteile zu vermeiden, besteht das Fällbad in einer besonders bevorzugten Ausführungsform der vorliegenden Erfindung aus einen Strömungskanal, der typischerweise Teil eines Kreislaufes mit Kühlung, Pumpe und Abtrennvorrichtung ist. Ein Fließschema der gesamten Anlage ist in Abbildung 1 wiedergegeben. Um im Strömungskanal eine gleichförmige Strömung zu erzielen, hat es sich als vorteilhaft erwiesen, den Einlassstrom über ein Maschensieb zu beruhigen. Die Vertropfung erfolgt danach in eine sehr gleichförmige Strömung, die die Tropfen so schnell aus der Eintropfzone wegfördert, dass sie nicht von nachfolgenden Tropfen getroffen werden und verkleben können. Durch die gezielte Wahl der Strömungsführung werden die Tropfen so bewegt, dass sie gleichmäßig mit Falllösung benetzt werden, was dazu führt, dass die Hüllbildung durch Reaktion der polyanionischen mit der polykationischen Verbindung unmittelbar und gleichmäßig um den gesamten Tropfen erfolgt und kugelförmige Aggregate gebildet werden.

Solange die Kapseln noch nicht völlig ausgehärtet sind, schwimmen sie auf; mit fortschreitender Aushärtung sedimentieren sie. In einer weiteren bevorzugten Ausführungsform der Erfindung wird die Strömung durch eine Reihe von hintereinandergeschalteten Wehren so geführt, dass sich ein gleichbleibendes Niveau einstellt und sowohl aufschwimmende als auch absinkende Kapseln dergestalt abtransportiert werden, dass sie während des Vorgangs des Aushärtens nicht zusammenstoßen. Düsendurchsatz und Länge des Strömungskanals können vom Fachmann routinemäßig so aufeinander abgestimmt werden, dass die Kapseln im Strömungskanal aushärten. Sie können dann ohne Zusammenkleben aus der zirkulierenden Falllösung, beispielsweise durch Absieben oder mit Hilfe eines Hydrozyklons abgetrennt werden. Im kontinuierlichen Betrieb lassen sich auf diese Weise Durchsätze im Bereich von 1 bis 3 kg/h/Düse - bezogen auf die Menge der gebildeten Makrokapseln - einstellen.

### Polyanionverbindungen

Die Verkapselung erfolgt wie schon erläutert durch Grenzflächenreaktion zwischen den gegensätzlich geladenen polyionischen Verbindungen. Die Reaktion ist spontan und vollständig, wobei mit steigender Reaktionszeit die Hülle um die Tropfen dicker wird. Im Bereich ausreichender mechanischer Festigkeit lassen sich problemlos Kapselwandstärken im Bereich von 10 bis 70 µm erzielen. Dabei ist es grundsätzlich unerheblich, ob die polyanionische Verbindung zusammen mit dem Wirkstoff in das die Polykationverbindung enthaltende Fällbad getropft wird oder umgekehrt. Als Polyanionverbindungen eignen sich insbesondere die Salze der Alginsäure. Bei der Alginsäure handelt es sich um ein Gemisch carboxylgruppenhaltiger Polysaccharide mit folgendem idealisierten Monomerbaustein:

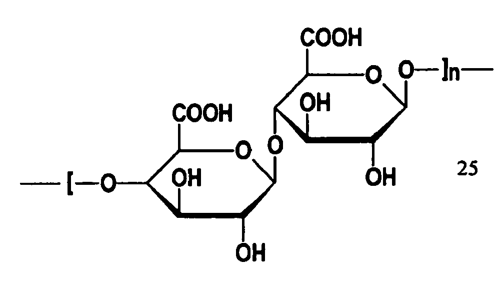

Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate. In einer alternativen Ausführungsform der Erfindung kommen für diesen Zweck jedoch auch anionische Chitosanderivate, wie z.B. Carboxylierungs- und vor allem Succinylierungsprodukte in Frage. Alternativ kommen auch Poly(meth)acrylate mit durchschnittlichen Molekulargewichten im Bereich von 5.000 bis 50.000 Dalton sowie die verschiedenen Carboxymethylcellulosen in Frage. Anstelle der anionischen Polymeren können für die Ausbildung der Hüllmembran auch anionische Tenside oder niedermolekulare anorganische Salze, wie beispielsweise Pyrophosphate eingesetzt werden. Üblicherweise setzt man die polyanionischen Verbindungen in Form wässriger 0,5 bis 1,5 Gew.-%iger Lösungen ein.

### Polykationverbindungen

Als Polykationverbindungen kommen mehrwertige wasserlösliche Metallsalze, insbesondere Calciumchlorid, sowie Kationpolymere, insbesondere Chitosan in Frage. Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

In einer besonderen Ausführungsform der Erfindung kommen als polykationische Verbindungen Chitosane in Betracht. Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

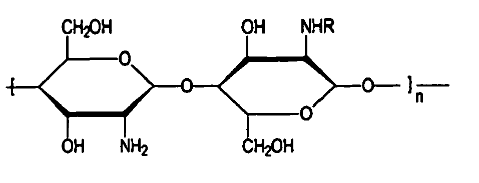

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, wie die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt. In einer besonders vorteilhaften Ausführungsform der vorliegenden Erfindung werden als Polykationverbindungen Mischungen von Calciumchlorid und Chitosan, vorzugsweise im Gewichtsverhältnis 20 : 80 bis 80 : 20 eingesetzt; auf diese Weise werden sehr beständige und doch flexible Hüllen erhalten. Üblicherweise setzt man auch die polykationischen Verbindungen in Form wässriger 0,5 bis 1,5 Gew.-%iger Lösungen ein.

### Wirkstoffe

Die Auswahl der zu verkapselnden Wirkstoffe ist im Sinne der Erfindung unkritisch und richtet sich ausschließlich nach der Aufgabenstellung. Typische Beispiele sind Oleochemikalien, Katalysatoren, Tenside, Enzyme, Phase changing materials (PCMs), Ölkörper, biogene Wirkstoffe, Antioxidantien, UV-Filter, Antischuppenmittel, Parfümöle und Aromen, Emulgatoren, Farbstoffe bzw. Farbpigmente sowie vollständige kosmetische oder pharmazeutische Emulsionen. In der Regel werden die Wirkstoffe in Mengen von 0,5 bis 70, vorzugsweise 1 bis 35 Gew.-% - bezogen auf die wässrigen Lösungen der polyanionischen bzw. polykationischen Verbindungen - eingesetzt. Typische Beispiele werden im Folgenden erläutert:

### Oleochemikalien

Geeignete Oleochemikalien sind Triglyceride, Partialglyceride, Fettsäuren, Fettsäureniedrigalkylester sowie Fettalkohole.

### Tenside

Als oberflächenaktive Stoffe können anionische, nichtionische, kationische und/oder amphotere bzw. zwitterionische Tenside verkapselt werden. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid (ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Enzyme

Als Enzyme kommen insbesondere solche aus der Klasse der Hydrolasen, wie der Proteasen, Esterasen, Lipasen bzw. lipolytisch wirkenden Enzyme, Amylasen, Cellulasen bzw. andere Glykosylhydrolasen und Gemische der genannten Enzyme in Frage. Alle diese Hydrolasen tragen in der Wäsche zur Entfernung von Verfleckungen, wie protein-, fett- oder stärkehaltigen Verfleckungen, und Vergrauungen bei. Cellulasen und andere Glykosylhydrolasen können durch das Entfernen von Pilling und Mikrofibrillen zur Farberhaltung und zur Erhöhung der Weichheit des Textils beitragen. Zur Bleiche bzw. zur Hemmung der Farbübertragung können auch Oxidoreduktasen eingesetzt werden. Besonders gut geeignet sind aus Bakterienstämmen oder Pilzen, wie Bacillus subtilis, Bacillus licheniformis, Streptomyces griseus und Humicola insolens gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase bzw. lipolytisch wirkenden Enzymen oder Protease und Cellulase oder aus Cellulase und Lipase bzw. lipolytisch wirkenden Enzymen oder aus Protease, Amylase und Lipase bzw. lipolytisch wirkenden Enzymen oder Protease, Lipase bzw. lipolytisch wirkenden Enzymen und Cellulase, insbesondere jedoch Protease- und/oder Lipase-haltige Mischungen bzw. Mischungen mit lipolytisch wirkenden Enzymen von besonderem Interesse. Beispiele für derartige lipolytisch wirkende Enzyme sind die bekannten Cutinasen. Auch Peroxidasen oder O-xidasen haben sich in einigen Fällen als geeignet erwiesen. Zu den geeigneten Amylasen zählen insbesondere α-Amylasen, Iso-Amylasen, Pullulanasen und Pektinasen. Als Cellulasen werden vorzugsweise Cellobiohydrolasen, Endoglucanasen und β-Glucosidasen, die auch Cellobiasen genannt werden, bzw. Mischungen aus diesen eingesetzt. Da sich die verschiedenen Cellulase-Typen durch ihre CMCase- und Avicelase-Aktivitäten unterscheiden, können durch gezielte Mischungen der Cellulasen die gewünschten Aktivitäten eingestellt werden.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂ - Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂ - Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Dihydroxyaceton, Arbutin, Ferulasäure, Kojisäure, Cumarinsäure und Ascorbinsäure (Vitamin C), Aminosäuren, Ceramide, Pseudoceramide, essentielle Öle, Pflanzenextrakte, wie z.B. Prunusextrakt, Bambaranussextrakt und Vitaminkomplexe zu verstehen.

### Antioxidantien

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µ mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### UV-Lichtschutzfilter

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
o 3-Benzylidencampher bzw. 3-Benzylidennorcampher und dessen Derivate, z.B. 3-(4-Methylbenzyliden)campher beschrieben;
o 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)
benzoesäureamylester;
o Ester der Zimtsäure, vorzugsweise 4-Methoxyzimtsäure-2-ethylhexylester,4-Methoxy-zimtsäurepropylester, 4-Methoxyzimtsäureisoamylester 2-Cyano-3,3-phenylzimtsäure-2-ethylhexylester (Octocrylene);
o Ester der Salicylsäure, vorzugsweise Salicylsäure-2-ethylhexylester, Salicylsäure-4-iso-propylbenzylester, Salicylsäurehomomenthylester;
o Derivate des Benzophenons, vorzugsweise 2-Hydroxy-4-methoxybenzophenon,2-Hydroxy-4-methoxy-4' -methylbenzophenon, 2,2'-Dihydroxy-4-methoxybenzophenon;
o Ester der Benzalmalonsäure, vorzugsweise 4-Methoxybenzmalonsäuredi-2-ethylhexylester;
o Triazinderivate, wie z.B. 2,4,6-Trianilino-(p-carbo-2'-ethyl-l'-hexyloxy)-1,3,5-triazin und Octyl Triazon oder Dioctyl Butamido Triazone (Uvasorb® HEB);
o Propan-1,3-dione, wie z.B. 1-(4-tert.Butylphenyl)-3-(4'methoxyphenyl)propan-1,3-dion;
o Ketotricyclo(5.2.1.0)decan-Derivate.

Als wasserlösliche Substanzen kommen in Frage:
o 2-Phenylbenzimidazol-5-sulfonsäure und deren Alkali-, Erdalkali-, Ammonium-, Alkylammonium-, Alkanolammonium- und Glucammoniumsalze;
o Sulfonsäurederivate von Benzophenonen, vorzugsweise 2-Hydroxy-4-methoxybenzophenon-5-sulfonsäure und ihre Salze;
o Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

### Antischuppenwirkstoffe

Typische Beispiele für Antischuppenwirkstoffe sind Pirocton, Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl)r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfelteer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt. Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe und Farbpigmente

Als Farbstoffe können die für kosmetische Zwecke geeigneten und zugelassenen Substanzen verwendet werden, wie sie beispielsweise in der Publikation **"**Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

### Emulgatoren

In einigen Fällen werden die zu verkapselnden Wirkstoffe in Wasser unlöslich sein. In diesem Fall kann es empfehlenswert sein, den Lösungen Emulgatoren bzw. Dispergatoren zuzusetzen, wobei deren Einsatzmenge 0,1 bis 5, vorzugsweise 0,5 1 Gew.-% bezogen auf die wässrigen Zubereitungen betragen kann. Als Emulgatoren kommen beispielsweise nichtionogene Tenside aus mindestens einer der folgenden Gruppen in Frage:
o Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
o Alkyl- und/oder Alkenyloligoglykoside mit 8 bis 22 Kohlenstoffatomen im Alk(en)ylrest und deren ethoxylierte Analoga;
o Anlagerungsprodukte von 1 bis 15 Mol Ethylenoxid an Ricinusöl und/oder gehärtetes Ricinusöl;
o Anlagerungsprodukte von 15 bis 60 Mol Ethylenoxid an Ricinusöl und/oder gehärtetes Ricinusöl;
o Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
o Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
o Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
o Mono-, Di- und Trialkylphosphate sowie Mono-, Di- und/oder Tri-PEGalkylphosphate und deren Salze;
o Wollwachsalkohole;
o Polysiloxan-Polyalkyl-Polyether-Copolymere bzw. entsprechende Derivate;
o Block-Copolymere z.B. Polyethylenglycol-30 Dipolyhydroxystearate;
o Polymeremulgatoren, z.B. Pemulen-Typen (TR-1,TR-2) von Goodrich;
o Polyalkylenglycole sowie
o Glycerincarbonat.

### Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

### Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

### Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

### Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

### Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

### Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Hilfs- und Zusatzstoffe

Typische Beispiele für Hilfs- und Zusatzstoffe, welche den Makrokapseln hinzugefügt werden, sind Viskositätsregulatoren und pH-Additive. Zur Steigerung der Viskosität werden vorzugsweise solche Stoffe eingesetzt, welche die Eigenschaft zeigen in wässriger Lösung bei Temperaturen oberhalb von 40 °C Gele zu bilden. Typische Beispiele hierfür sind Heteropolysaccharide und Proteine. Als thermogelierende Heteropolysaccharide kommen vorzugsweise Agarosen in Frage, welche in Form des aus Rotalgen zu gewinnenden Agar-Agar auch zusammen mit bis zu 30 Gew.-% nicht-gelbildenden Agaropektinen vorliegen können. Hauptbestandteil der Agarosen sind lineare Polysaccharide aus D-Galaktose und 3,6-Anhydro-L-galaktose, die alternierend β-1,3- und β-1,4-glykosidisch verknüpft sind. Die Heteropolysaccharide besitzen vorzugsweise ein Molekulargewicht im Bereich von 110.000 bis 160.000 und sind sowohl farb- als auch geschmacklos. Als Alternativen kommen Pektine, Xanthane (auch Xanthan Gum) sowie deren Mischungen in Frage. Es sind weiterhin solche Typen bevorzugt, die noch in 1-Gew.-%iger wässriger Lösung Gele bilden, die nicht unterhalb von 80 °C schmelzen und sich bereits oberhalb von 40 °C wieder verfestigen. Aus der Gruppe der thermogelierenden Proteine seien exemplarisch die verschiedenen Gelatine-Typen genannt. Daneben können auch Polyole, speziell Glycerin, sowie Polyalkylenglycole, insbesondere Polyethylenglycole mit Molekulargewichten im Bereich von 100 bis 1.000 eingesetzt werden. Die Einstellung des pH-Wertes kann beispielsweise mit organischen Säuren wie Essig- oder Zitronensäure erfolgen. Die Einsatzmenge der Hilfs- und Zusatzstoffe kann bezogen auf die wässrigen Zubereitungen 0,1 bis 2 und vorzugsweise 0,5 bis 1 Gew.-% betragen.

### Gewerbliche Anwendbarkeit

Die neuen Makrokapseln können die unterschiedlichsten Wirkstoffe enthaltend; entsprechend vielfältig ist ihr Anwendungsgebiet. Sie eignen sich daher beispielsweise zur Herstellung von
o kosmetischen Zubereitungen, speziell solchen zur Pflege von Haut und Haaren, wie beispielsweise Haarshampoos, Haarlotionen, Schaumbädern, Duschbädern, Cremes, Gelen, Lotionen, alkoholischen und wässrig/alkoholisches Lösungen, Emulsionen, Wachs/ Fett-Massen und/oder Stiftpräparaten;
o pharmazeutischen Zubereitungen;
o Nahrungsmittelzusatzstoffen;
o Zubereitungen für die Tierernährung;
o Zubereitungen für die Ausrüstung von Fasern, Garnen und textilen Endprodukten,
wobei deren Einsatzmenge jeweils 0,1 bis 30, vorzugsweise 1 bis 15 Gew.-% - bezogen auf die Endzubereitung - betragen kann.

### Beispiele

### Beispiel 1

Eine blau gefärbte wässrige Lösung enthaltend 5 Gew.-% Glycerin, 0,5 Gew.-% Natriumalginat (Texamid® 558 P, Cognis) und 0,5 Gew.-% eines blauen löslichen Farbpigmentes wurde bei pH = 5,4 in einer Mini-Droppo-Anlage der Firma Rieter Automatik GmbH in ein wässriges Fällbad bestehend aus einer 1 Gew.-%igen Calciumchloridlösung vertropft; der Durchmesser der Lochdüse betrug 0,8 mm. Das Fällbad wurde durch einen Kanal mit den Abmessungen 63 x 50 x 186 mm kontinuierlich umgewälzt. Unmittelbar nach dem Eintauchen der Tropfen in das Fällbad bildete sich eine Membran, die die Tropfen umhüllte. Die so erhaltenen nahezu kugelförmigen Makrokapseln wurden nach einer Verweilzeit von weniger als 5 s aus dem strömenden Fällbad abgesiebt. Ein Zusammenkleben der Kapseln wurde nicht beobachtet. Während einer Vertropfungszeit von 1 h wurden 2,6 kg Makrokapseln erhalten, von denen 95 % einen mittleren Durchmesser von 1,2 bis 1,6 mm aufweisen. Das Fällbad blieb klar und verfärbte sich nicht, was zeigt, dass der Einschluss der Farbpigmente quantitativ war.

### Beispiel 2

Beispiel 1 wurde wiederholt, jedoch durch eine Lochdüse mit einem Durchmesser von 0,3 mm vertropft. Der Durchsatz betrug 1,1 kg/h. Es wurden vollständig kugelförmige Makrokapseln erhalten, von denen 99 % einen mittleren Durchmesser von 0,5 bis 0,7 mm aufwiesen.

### Beispiel 3

Beispiel 1 wurde mit einer orangefarbigen wässrigen Dispersion enthaltend 5 Gew.-% Mineralöl, 0,5 Gew.-% Natriumalginat, 1 Gew.-% Agar Agar und 0,5 Gew.-% lösliches orangefarbenes Pigment wiederholt. Die auf 50 °C vorgewärmte Dispersion wurde bei pH = 5,3 wiederum in eine wässrige Calciumchloridlösung vertropft (Düsendurchmesser 0,8 mm). 98 % der so erhaltenen Makrokapseln zeigten einen mittleren Durchmesser von 1,2 bis 1,6 mm. Das Fällbad blieb farblos; es bildete sich kein Ölfilm.

### Beispiel 4

Beispiel 1 wurde mit einer hellblau gefärbten wässrigen Dispersion enthaltend 5 Gew.-% Glycerin, 0,5 Gew.-% Natriumalginat, 1 Gew.-% Agar Agar, 0,5 Gew.-% löslichem hellblauem Pigment sowie 1 Gew.-% unlöslichem Mica Pigment wiederholt. Die auf 50 °C vorgewärmte Dispersion wurde bei pH = 5,3 wiederum in eine wässrige Calciumchloridlösung vertropft (Düsendurchmesser 0,8 mm). 98 % der so erhaltenen Makrokapseln zeigten einen mittleren Durchmesser von 1,2 bis 1,6 mm. Das Fällbad blieb farblos; eine Verstopfung der Düsen wurde nicht festgestellt.

### Beispiel 5

Beispiel 1 wurde mit einer wässrigen Dispersion enthaltend 5 Gew.-% Glycerin, 0,5 Gew.-% Natriumalginat, 1 Gew.-% Agar Agar sowie 1 Gew.-% Retinol wiederholt. Die auf 50 °C vorgewärmte Lösung wurde bei pH = 5,3 wiederum in eine wässrige Calciumchloridlösung vertropft (Düsendurchmesser 0,8 mm). 98 % der so erhaltenen Makrokapseln zeigten einen mittleren Durchmesser von 1,2 bis 1,6 mm.

### Beispiel 6

Beispiel 1 wurde mit einer wässrigen Dispersion enthaltend 20 Gew.-% Capric Caprylic Triglycerides (Myritol® 312, Cognis), 1 Gew.-% Natriumalginat und 2 Gew.-% Polyethylenglycol (MW = 200) wiederholt. Die auf 50 °C vorgewärmte Dispersion wurde bei pH = 5,3 in eine wässrige Lösung enthaltend 0,7 Gew.-% Calciumchlorid und 0,3 Gew.-% Chitosan (Hydagen® CMF, Cognis) vertropft (Düsendurchmesser 0,8 mm). 100 % der so erhaltenen Makrokapseln zeigten einen mittleren Durchmesser von 1,2 bis 1,6 mm. Es wurde eine quantitative Umhüllung der Öltröpfchen beobachtet; das Fällbad blieb klar. Die elastischen, ölgefüllten Makrokapseln waren in wässriger Lösung lagerstabil.

### Beispiel 7

Beispiel 1 wurde mit einer bei 50 °C hergestellten wässrigen Dispersion enthaltend 30 Gew.-% Laurylalkohol, 0,7 Gew.-% Natriumalginat, 5 Gew.-% Polyethylenglycol (MW =200) und 0,25 Gew.-% Ceteareth-20 wiederholt. Die auf 50 °C vorgewärmte Dispersion wurde bei pH = 5,3 in eine wässrige Lösung enthaltend 1,0 Gew.-% Calciumchlorid und 5 Gew.-% Polyethylenglycol (MW =200) vertropft (Düsendurchmesser 0,8 mm). 100 % der so erhaltenen Makrokapseln zeigten einen mittleren Durchmesser von 1 bis 2 mm. Die mit einem Sieb abfiltrierten Makrokapseln wurden mit Wasser gewaschen. Die Makrokapseln bildeten Makrosponches, die sich drücken und kneten ließen, ohne aufzuspringen.

Die nachfolgende Tabelle enthält eine Reihe von Formulierungsbeispielen.

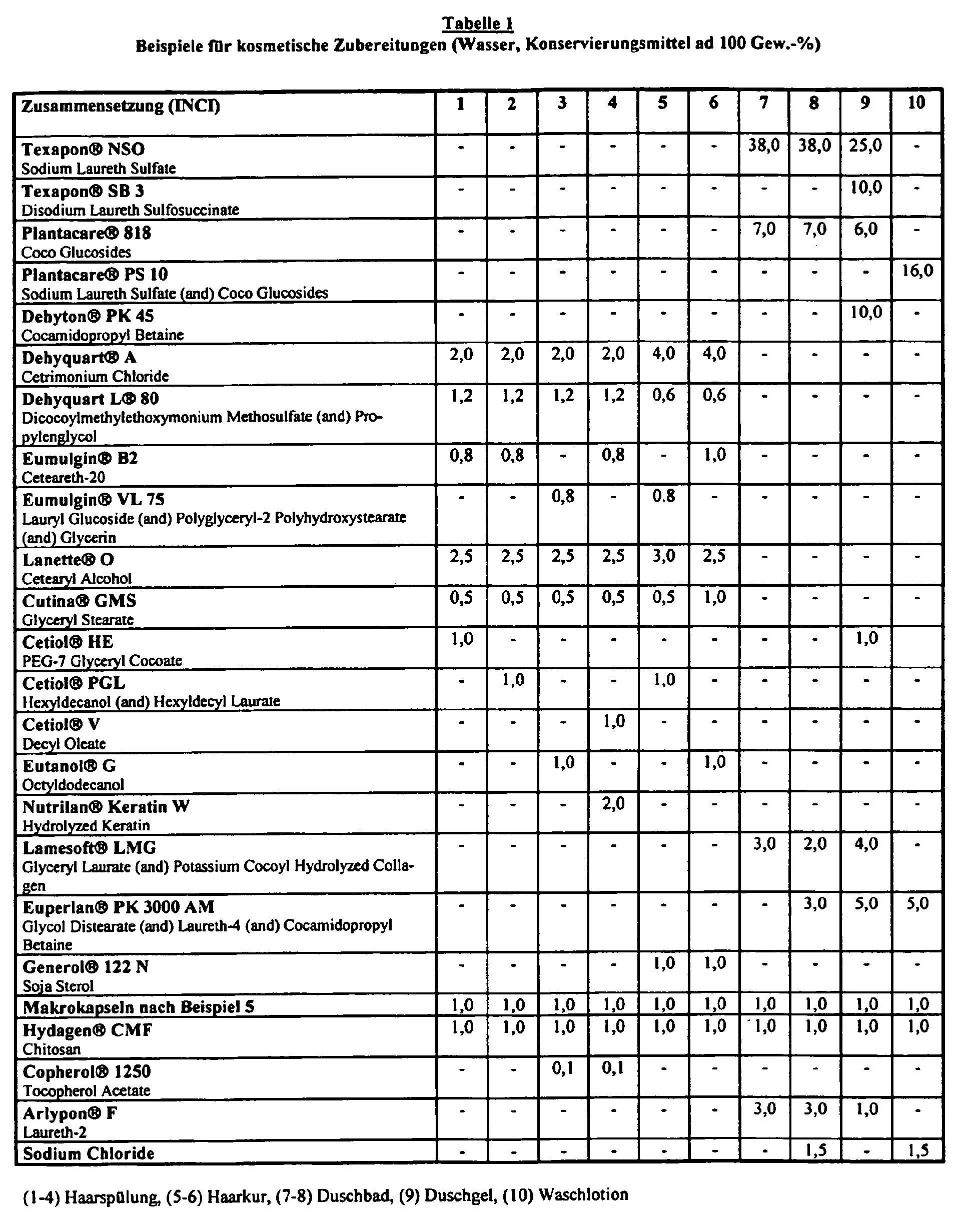

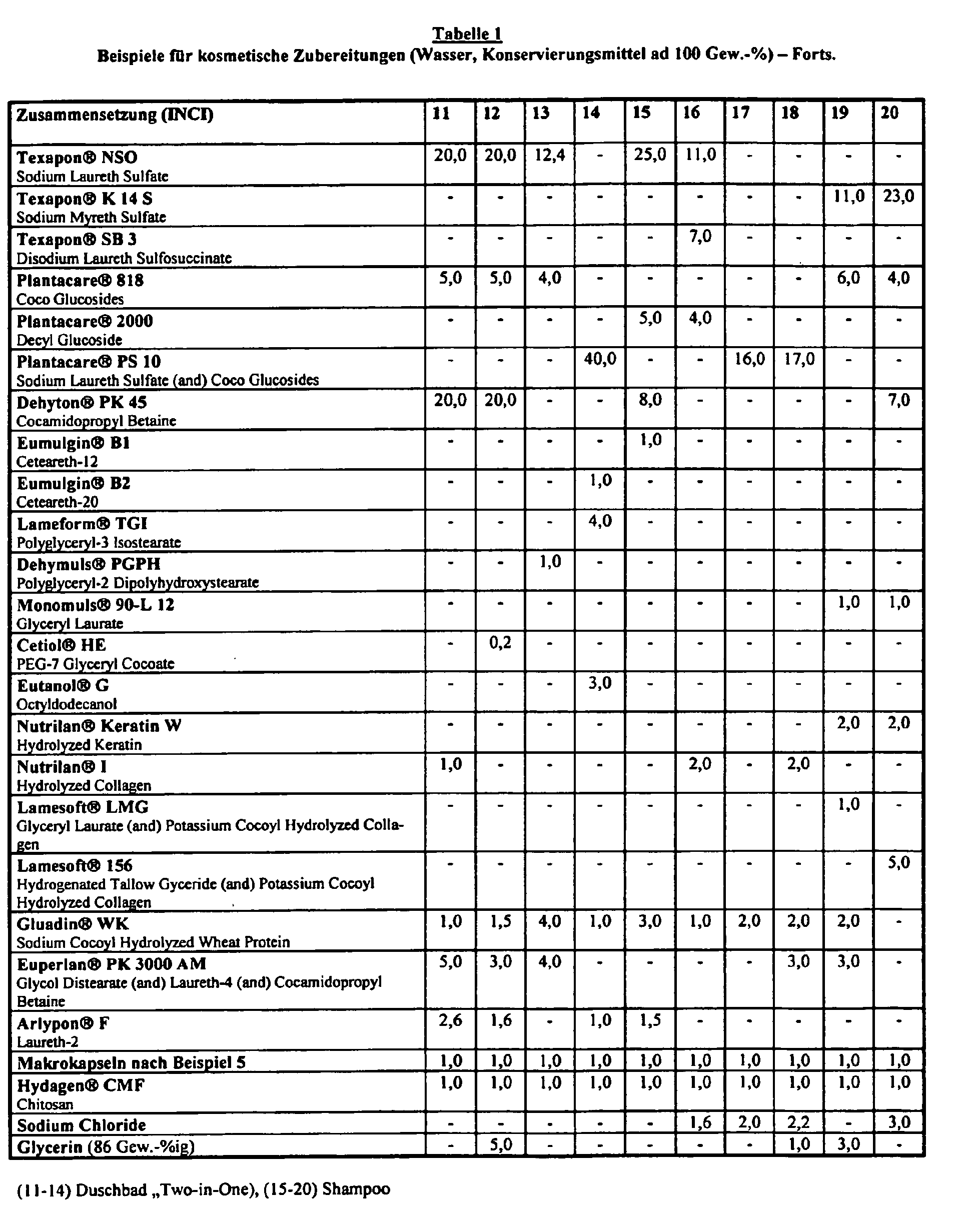

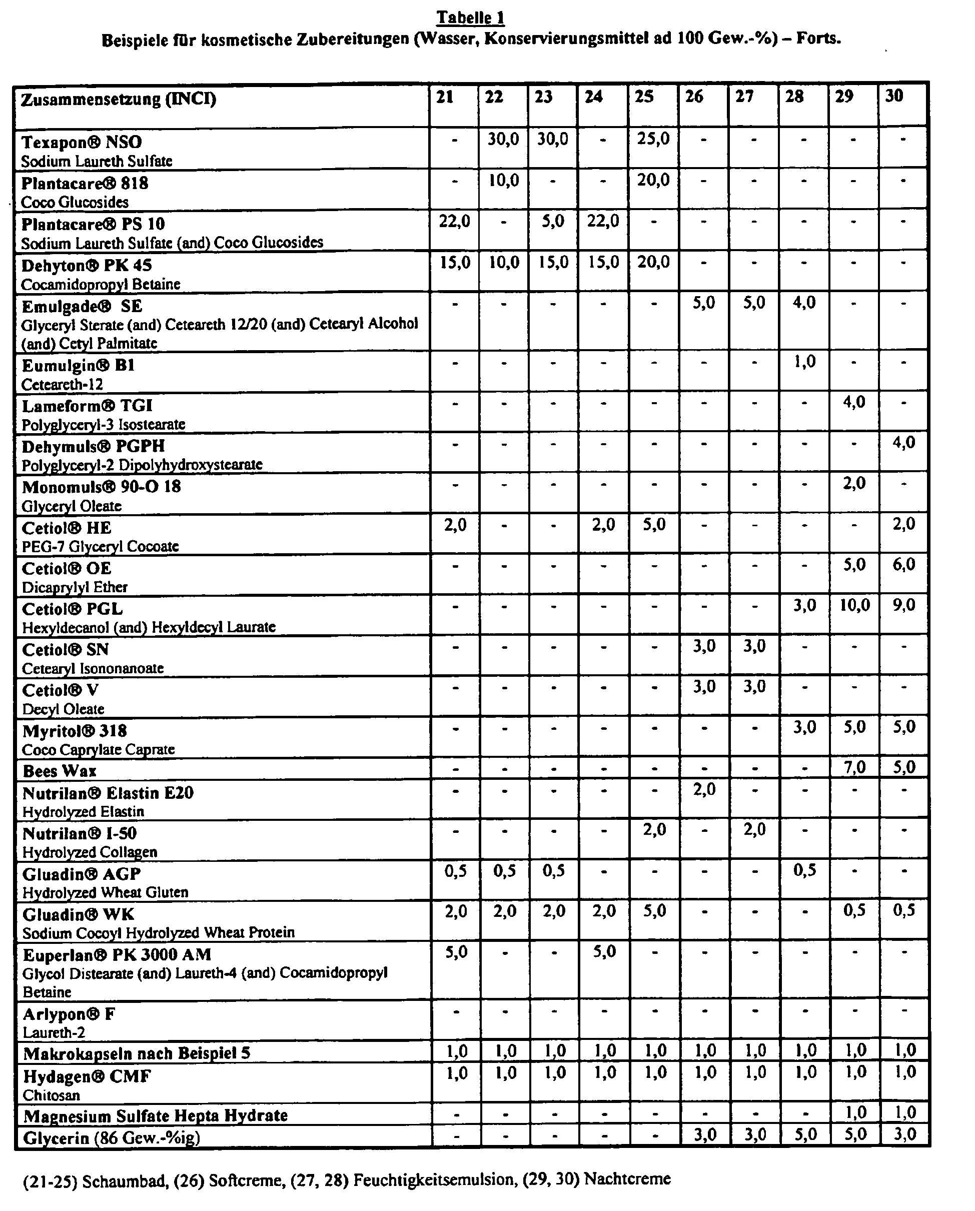

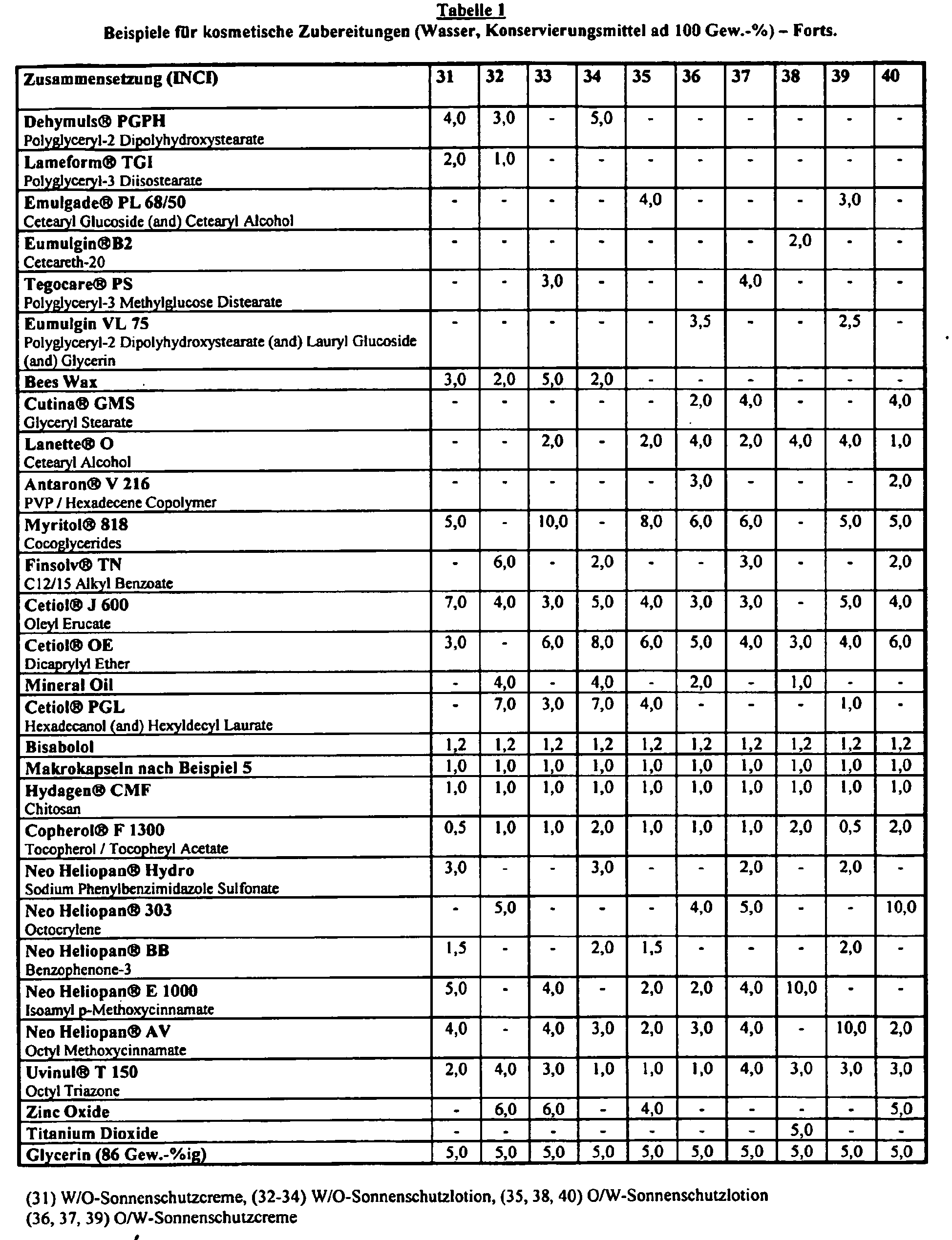

## Patentansprüche

1. Verfahren zur kontinuierlichen Herstellung von Makrokapseln, bestehend aus einer Hüllmembran und einem darin eingeschlossenen Wirkstoff, bei dem man
(a1) einer wässrigen Zubereitung enthaltend mindestens eine polyanionische Verbindung und mindestens einen Wirkstoff mit Hilfe einer oszillierenden Membran eine Schwingung aufprägt und durch mindestens eine Düse vertropft und
(a2) die so erhaltenen Tropfen in ein Fällbad enthaltend mindestens eine polykationische Verbindung einbringt,
oder
(b1) einer wässrigen Zubereitung enthaltend mindestens eine polykationische Verbindung und mindestens einen Wirkstoff mit Hilfe einer oszillierenden Membran eine Schwingung aufprägt und durch mindestens eine Düse vertropft und
(b2) die so erhaltenen Tropfen in ein Fällbad enthaltend mindestens eine polyanionische Verbindung einbringt.

2. Verfahren nach Anspruch 1, **dadurch gekennzeichnet, dass** die Membran mit einer Frequenz von 100 bis 1.000 Hz schwingt.

3. Verfahren nach den Ansprüchen 1 und/oder 2, **dadurch gekennzeichnet, dass** die Vertropfung durch eine Einzeldüse oder eine Düsenplatte mit 10 bis 500 Öffnungen erfolgt.

4. Verfahren nach mindestens einem der Ansprüche 1 bis 3, **dadurch gekennzeichnet, dass** man Einzeldüsen einsetzt, die eine Öffnung mit einem Durchmesser im Bereich von 0,2 bis 2 mm aufweisen.

5. Verfahren nach mindestens einem der Ansprüche 1 bis 4, **dadurch gekennzeichnet, dass** man Mehrstoffdüsen mit einem Kernrohr und einem koaxial angeordneten Außenrohr einsetzt, die Öffnungen mit einem Durchmesser im Bereich von 0,2 bis 2 mm aufweisen.

6. Verfahren nach mindestens einem der Ansprüche 1 bis 5, **dadurch gekennzeichnet, dass** man Düsenplatten einsetzt, die Öffnungen mit einem Durchmesser im Bereich von 0,3 bis 1 mm aufweisen.

7. Verfahren nach mindestens einem der Ansprüche 1 bis 6, **dadurch gekennzeichnet, dass** man als Fällbad einen Strömungskanal einsetzt.

8. Verfahren nach mindestens einem der Ansprüche 1 bis 7, **dadurch gekennzeichnet, dass** man die Makrokapseln durch Sieben aus dem Fällbad entfernt.

9. Verfahren nach mindestens einem der Ansprüche 1 bis 8, **dadurch gekennzeichnet, dass** man einen Durchsatz im Bereich von 1 bis 3 kg/h - bezogen auf die Menge der gebildeten Makrokapseln - einstellt.

10. Verfahren nach mindestens einem der Ansprüche 1 bis 9, **dadurch gekennzeichnet, dass** man Polyanionverbindungen einsetzt, die ausgewählt sind aus der Gruppe, die gebildet wird von Alginsäure und/oder deren Salzen, anionischen Chitosanderivaten, Poly(meth)acrylaten, Aniontensiden und niedermolekularen anionischen Salzen.

11. Verfahren nach mindestens einem der Ansprüche 1 bis 10, **dadurch gekennzeichnet, dass** man die Polyanionverbindungen in Form wässriger 0,5 bis 1,5 Gew.-%iger Lösungen einsetzt.

12. Verfahren nach mindestens einem der Ansprüche 1 bis 11, **dadurch gekennzeichnet, dass** man Polykationverbindungen einsetzt, die ausgewählt sind aus der Gruppe, die gebildet wird von wasserlöslichen mehrwertigen Metallsalzen und Kationpolymeren.

13. Verfahren nach mindestens einem der Ansprüche 1 bis 12, **dadurch gekennzeichnet, dass** man die Polykationverbindungen in Form 0,5 bis 1,5 Gew.-%iger wässriger Lösungen einsetzt.

14. Verfahren nach mindestens einem der Ansprüche 1 bis 13, **dadurch gekennzeichnet, dass** man Wirkstoffe einsetzt, die ausgewählt sind aus der Gruppe, die gebildet wird von Oleochemikalien, Katalysatoren, Tensiden, Enzymen, Ölkörpern, biogenen Wirkstoffen, Antioxidantien, UV-Lichtschutzfiltern, Antischuppenwirkstoffen, Parfümölen und Aromen, Farbstoffen bzw. Farbpigmenten sowie kosmetischen oder pharmazeutischen Emulsionen.

15. Verfahren nach mindestens einem der Ansprüche 1 bis 14, **dadurch gekennzeichnet, dass** man die Wirkstoffe in Mengen von 0,5 bis 25 Gew.-% - bezogen auf die wässrigen Lösungen der polyanionischen bzw. polykationischen Verbindungen einsetzt.

16. Verfahren nach mindestens einem der Ansprüche 1 bis 15, **dadurch gekennzeichnet, dass** man die polyanionischen bzw. polykationischen Verbindungen und die Wirkstoffe zusammen mit Emulgatoren bzw. Dispergatoren einsetzt.

17. Verfahren nach mindestens einem der Ansprüche 1 bis 16, **dadurch gekennzeichnet, dass** man die polyanionischen bzw. polykationischen Verbindungen und die Wirkstoffe zusammen mit Viskositätsregulatoren und/oder pH-Additiven einsetzt.

## Claims

1. Process for the continuous preparation of macrocapsules consisting of a shell membrane and an active ingredient enclosed therein, in which
(a1) oscillation is brought about of an aqueous preparation comprising at least one polyanionic compound and at least one active ingredient using an oscillating membrane, and is dropped through at least one nozzle and
(a2) the drops obtained in this way are introduced into a precipitation bath comprising at least one polycationic compound,
or
(b1) oscillation is brought about of an aqueous preparation comprising at least one polycationic compound and at least one active ingredient using an oscillating membrane, and the preparation is dropped through at least one nozzle and
(b2) the drops obtained in this way are introduced into a precipitation bath comprising at least one polyanionic compound.

2. Process according to Claim 1, **characterized in that** the membrane oscillates at a frequency of from 100 to 1000 Hz.

3. Process according to Claims 1 and/or 2, **characterized in that** the dropping takes place through an individual nozzle or a nozzle plate having 10 to 500 orifices.

4. Process according to at least one of Claims 1 to 3, **characterized in that** individual nozzles are used which have an orifice with a diameter in the range from 0.2 to 2 mm.

5. Process according to at least one of Claims 1 to 4, **characterized in that** multicomponent nozzles with an inner pipe and a coaxially arranged outer pipe are used which have orifices with a diameter in the range from 0.2 to 2 mm.

6. Process according to at least one of Claims 1 to 5, **characterized in that** nozzle plates are used which have orifices with a diameter in the range from 0.3 to 1 mm.

7. Process according to at least one of Claims 1 to 6, **characterized in that** the precipitation bath used is a flow channel.

8. Process according to at least one of Claims 1 to 7, **characterized in that** the macrocapsules are removed from the precipitation bath by sieving.

9. Process according to at least one of Claims 1 to 8, **characterized in that** a throughput in the range from 1 to 3 kg/h, based on the amount of macrocapsules formed, is set.

10. Process according to at least one of Claims 1 to 9, **characterized in that** polyanionic compounds are used which are chosen from the group consisting of alginic acid and/or salts thereof, anionic chitosan derivatives, poly(meth)acrylates, anionic surfactants and low molecular weight anionic salts.

11. Process according to at least one of Claims 1 to 10, **characterized in that** the polyanionic compounds are used in the form of aqueous 0.5 to 1.5% strength by weight solutions.

12. Process according to at least one of Claims 1 to 11, **characterized in that** polycationic compounds are used which are chosen from the group consisting of water-soluble polyvalent metal salts and cationic polymers.

13. Process according to at least one of Claims 1 to 12, **characterized in that** the polycationic compounds are used in the form of 0.5 to 1.5% strength by weight aqueous solutions.

14. Process according to at least one of Claims 1 to 13, **characterized in that** active ingredients are used which are chosen from the group consisting of oleochemicals, catalysts, surfactants, enzymes, oily bodies, biogenic active ingredients, antioxidants, UV light protection filters, antidandruff active ingredients, perfume oils and aromas, dyes or colour pigments, and cosmetic or pharmaceutical emulsions.

15. Process according to at least one of Claims 1 to 14, **characterized in that** the active ingredients are used in amounts of from 0.5 to 25% by weight, based on the aqueous solutions of the polyanionic or polycationic compounds.

16. Process according to at least one of Claims 1 to 15, **characterized in that** the polyanionic or polycationic compounds and the active ingredients are used together with emulsifiers or dispersants.

17. Process according to at least one of Claims 1 to 16, **characterized in that** the polyanionic or polycationic compounds and the active ingredients are used together with viscosity regulators and/or pH additives.

## Revendications

1. Procédé de préparation en continu de macrocapsules constituées d'une membrane enveloppe et d'un agent actif renfermé dans celle-ci, selon lequel :
(a1) on transmet une vibration à une préparation aqueuse contenant au moins un composé polyanionique et au moins un agent actif à l'aide d'une membrane oscillante et on la fait goutter à l'aide d'au moins une tuyère, et
(a2) on incorpore les gouttes ainsi obtenues dans un bain de précipitation contenant au moins un composé polycationique,
ou
(b1) on transmet une vibration à une préparation aqueuse contenant au moins un composé polycationique et au moins un agent actif à l'aide d'une membrane oscillante et on le fait goutter à l'aide d'au moins une tuyère, et
(a2) on incorpore les gouttes ainsi obtenues dans un bain de précipitation contenant au moins un composé polyanionique.

2. Procédé selon la revendication 1,
**caractérisé en ce que**
la membrane vibre à une fréquence de 100 à 1.000 Hz.

3. Procédé selon les revendications 1 et/ou 2,
**caractérisé en ce qu'**
on forme les gouttes à l'aide d'une tuyère individuelle ou d'un plateau-tuyères comportant de 10 à 500 orifices.

4. Procédé selon au moins l'une des revendications 1 à 3,
**caractérisé en ce qu'**
on utilise des tuyères individuelles comportant un orifice ayant un diamètre de l'ordre de 0,2 à 2 mm.

5. Procédé selon au moins l'une des revendications 1 à 4,
**caractérisé en ce qu'**
on utilise des tuyères à plusieurs constituants comportant un tube central et un tube extérieur disposé coaxialement, à celui-ci qui présentent des orifices ayant un diamètre de l'ordre de 0,2 à 2 mm.

6. Procédé selon au moins l'une des revendications 1 à 5,
**caractérisé en ce qu'**
on utilise des plaques-tuyères présentant des orifices ayant un diamètre de l'ordre de 0,3 à 1 mm.

7. Procédé selon au moins l'une des revendications 1 à 6,
**caractérisé en ce qu'**
on utilise un canal d'écoulement comme bain de précipitation.

8. Procédé selon au moins l'une des revendications 1 à 7,
**caractérisé en ce qu'**
on retire les macrocapsules du bain de précipitation par tamisage.

9. Procédé selon au moins l'une des revendications 1 à 8,
**caractérisé en ce qu'**
on règle un débit de l'ordre de 1 à 3 kg/h - par rapport à la quantité des macrocapsules formées.

10. Procédé selon au moins l'une des revendications 1 à 9,
**caractérisé en ce qu'**
on utilise des composés polyanioniques choisis dans le groupe formé d'acide alginique et/ou de ses sels, de dérivés anioniques de chitosane, de poly(méth)acrylates, de tensioactifs anioniques et de sels anioniques à faible poids moléculaire.

11. Procédé selon au moins l'une des revendications 1 à 10,
**caractérisé en ce qu'**
on utilise les composés polyanioniques sous forme de solutions aqueuses à 0,5 à 1,5 % en poids.

12. Procédé selon au moins l'une des revendications 1 à 11,
**caractérisé en ce qu'**
on utilise des composés polycationiques choisis dans le groupe formé de sels métalliques polyvalents hydrosolubles et de polymères cationiques.

13. Procédé selon au moins l'une des revendications 1 à 12,
**caractérisé en ce qu'**
on utilise les composés polycationiques sous forme de solutions aqueuses à 0,5 à 1,5 % en poids.

14. Procédé selon au moins l'une des revendications 1 à 13,
**caractérisé en ce qu'**
on utilise des agents actifs choisis dans le groupe formé de produits chimiques huileux, de catalyseurs, de tensioactifs, d'enzymes, de corps huileux, d'agents actifs biogènes, d'antioxydants, de filtres solaires anti-UV, d'agents antipelliculaires, d'huiles parfumées et d'arômes, de colorants ou de pigments colorés ainsi que d'émulsions cosmétiques ou pharmaceutiques.

15. Procédé selon au moins l'une des revendications 1 à 14,
**caractérisé en ce qu'**
on utilise les agents actifs en quantités de 0,5 à 25 % en poids par rapport aux solutions aqueuses de composés polyanioniques ou polycationiques.

16. Procédé selon au moins l'une des revendications 1 à 15,
**caractérisé en ce qu'**
on utilise les composés polyanioniques ou polycationiques et les agents actifs en combinaison avec des agents émulsifiants ou dispersants.

17. Procédé selon au moins l'une des revendications 1 à 16,
**caractérisé en ce qu'**
on utilise les composés polyanioniques ou polycationiques et les agents actifs en combinaison avec des régulateurs de viscosité et/ou des additifs de réglage du pH.
